# FASCICULE DE BREVET EUROPEEN

(11) **EP 2 356 208 B1**
(45) Date de publication et mention de la délivrance du brevet: **27.01.2016**
(21) Numéro de dépôt: 09759935.1
(22) Date de dépôt: 23.11.2009
(51) Int. Cl.: C12M 1/22

(54) **DISPOSITIF DE DISTRIBUTION D'UN PRODUIT DANS UNE BOÎTE DE PETRI**
VORRICHTUNG ZUR ABGABE EINES PRODUKTS IN EINE PETRISCHALE
DEVICE FOR DISPENSING A PRODUCT IN A PETRI DISH

(30) Priorité: 10.12.2008 FR 0858437
(43) Date de publication de la demande: 17.08.2011
(73) Titulaire: AES Chemunex, 35270 Combourg (FR)
(72) Inventeur: BRELIVET, Nicolas, F-22350 Saint Jouan De L'Isle (FR)
(74) Mandataire: Regimbeau
(86) Numéro de dépôt international: PCT/EP2009/065625
(87) Numéro de publication internationale: WO 2010/066562

(56) Documents cités:
- EP-A- 0 385 902
- FR-A- 2 433 752
- US-A- 3 704 568
- US-A- 3 844 896

## Description

L'invention concerne un dispositif et de distribution d'un produit prescrit, notamment de gélose, dans des boîtes de Petri.

Les boîtes de Petri sont de petites boîtes de contour circulaire, en matière plastique transparente, qui contiennent un produit nutritif tel qu'un milieu gélosé ; elles servent de milieu de développement pour des cultures contenant des micro-organismes et sont utilisées en grand nombre par les laboratoires d'analyse, notamment dans la recherche médicale et l'industrie.

Un dispositif de distribution selon le préambule de la revendication 1 est connu par le document EP-B-385 902. Ce dispositif comprend un plateau tournant pour amener successivement et pas-à-pas des boîtes ouvertes ayant leur couvercle maintenu écarté au-dessus du fond, d'une station distributrice de boîtes vides à une station de remplissage, puis de cette dernière à une station réceptrice de boîtes remplies, ce plateau étant composé de deux platines horizontales circulaires coaxiales et superposées, percées chacune d'une série d'ouvertures circulaires en correspondance l'une avec l'autre, les ouvertures de la platine supérieure ayant un diamètre plus petit que celui des couvercles mais légèrement plus grand que celui des fonds pour autoriser le passage des fonds en retenant les couvercles, un piston étant prévu pour transférer une boîte vide dans le plateau en mettant son fond dans la platine inférieure et en retenant son couvercle dans le plateau supérieur et un autre piston étant prévu pour faire remonter un fond ayant été rempli de la platine inférieure dans le couvercle retenu dans la platine supérieure.

Ce dispositif présente de nombreux inconvénients.

Il possède une cadence de remplissage de l'ordre d'une boîte toutes les 6 secondes selon ce qui est indiqué dans le document, ce qui est relativement faible.

En outre, le temps d'ouverture de chaque boîte de Petri est relativement long (environ 16 secondes pour un plateau tournant d'un cran toutes les 4 secondes).

Par conséquent, le risque de pollution de l'intérieur de la boîte par des particules indésirables est grand.

Enfin, pour pouvoir remplir le plus grand nombre possible de boîtes en un minimum de temps, le dispositif possède une hauteur relativement grande, ce qui engendre des difficulté d'accès en haut du carrousel pour charger les piles de boîtes vides et retirer les piles de boîtes remplies. Ce problème est d'autant plus gênant que les personnes se servant du dispositif sont le plus souvent des femmes, lesquelles, statistiquement, sont moins grandes que les hommes.

Le document FR-A-2 433 752 décrit un dispositif dans lequel les boîtes de Petri sont dépilées de la partie inférieure d'un carrousel vers une platine rotative à deux plaques superposées. Cette platine rotative comporte trois stations : une station de dépilement de boîtes de Petri, une station de remplissage par un tube et une station élévatrice par un moyen élévateur pour remonter la boîte remplie dans une autre pile du carrousel. Ainsi, ce dispositif est un dispositif de distribution d'un produit prescrit dans au moins une boîte de Petri, chaque boîte de Petri comportant un couvercle amovible apte à être disposé sur un fond moins large que celui-ci, le dispositif comportant sur un socle au moins des première et deuxième colonnes de guidage vertical de boîtes de Petri, aptes à recevoir respectivement des première et deuxième piles de boîtes de Petri à couvercle disposé sur le fond et aptes à être positionnées respectivement au-dessus d'un premier passage inférieur de boîtes de Petri et au-dessus d'un deuxième passage inférieur de boîtes de Petri, les premier et deuxième passages étant prévus sur une partie fixe par rapport au socle, un système de déplacement d'au moins une boîte de Petri de la première colonne à la deuxième colonne, comportant un organe de transfert ayant une ouverture supérieure de support du couvercle d'une boîte de Petri au-dessus d'une deuxième ouverture inférieure de support du fond d'une boîte de Petri, un moyen de distribution pour distribuer ledit produit au-dessus de l'ouverture inférieure dans le fond de la boîte de Petri sous l'ouverture supérieure, l'organe mobile de transfert étant apte à occuper une première position d'arrêt, où les ouvertures de support se trouvent sous le premier passage, et une deuxième position d'arrêt, où les ouvertures de support se trouvent sous le deuxième passage, un piston comportant un plateau situé sous le deuxième passage inférieur et apte à traverser les ouvertures pour faire remonter le couvercle et le fond de la boîte de Petri de leur ouverture respective de support dans la deuxième colonne, lorsque l'organe de transfert se trouve dans la deuxième position d'arrêt associée.

Ce dispositif présente également les inconvénients mentionnés ci-dessus.

Des dispositifs à convoyeur linéaire sont également connus.

Le document US-A-3 704 568 décrit un dispositif de remplissage de boîtes de Petri comportant un convoyeur exécutant un mouvement linéaire sous une pile de boîtes de Petri vides vers une partie fixe comportant deux rails supérieurs écartés l'un de l'autre d'une plus petite distance que deux rails inférieurs, le convoyeur comportant un plateau supérieur et un plateau inférieur. Dans ce dispositif, une boîte de Petri est amenée de la pile sur le plateau supérieur. Puis après un recul vers la gauche, le plateau est translaté vers la droite pour que les rails supérieurs retiennent le couvercle et que le fond de la boîte tombe sur les rails inférieurs. Le remplissage est alors effectué dans le fond ouvert. Puis une surface verticale avant du convoyeur pousse le couvercle et le fond rempli vers la gauche au-delà des rails supérieurs pour faire tomber le couvercle sur le fond et pousser la boîte sur une table de réception.

Par conséquent, ce dispositif ne produit pas de piles de boîtes de Petri remplies et il faut donc qu'un utilisateur empile les boîtes remplies. Par ailleurs, les boîtes vides doivent rencontrer un crochet pour soulever le couvercle au cours de la translation du convoyeur, ce qui exerce une force de butée sur les boîtes et risque de les abîmées. Chaque boîte remplie arrive sur une table de réception éloignée d'une distance égale à trois largeurs de boîtes par rapport à la pile de boîtes vides. Par conséquent, ce dispositif est également très encombrant.

D'autres dispositifs à convoyeur linéaire à bande sans fin sont connus par les documents US-A-4 468 914 et US-A-5 698 260.

Ces dispositifs sont encore plus encombrants.

L'invention vise à obtenir un dispositif palliant les inconvénients mentionnés ci-dessus par un dispositif fournissant une pile de boîtes de Petri remplies à partir d'une pile de boîtes de Petri vides, qui soit de faible encombrement, qui puisse avoir une cadence élevée de remplissage, une grande capacité de boîtes en évitant ou en diminuant les risques de contamination des boîtes au cours de leur ouverture.

A cet effet, un premier objet de l'invention est un dispositif de distribution d'un produit prescrit dans au moins une boîte de Petri, chaque boîte de Petri comportant un couvercle amovible apte à être disposé sur un fond moins large que celui-ci, le dispositif comportant sur un socle :
au moins des première et deuxième colonnes de guidage vertical de boîtes de Petri, aptes à recevoir respectivement des première et deuxième piles de boîtes de Petri à couvercle disposé sur le fond et aptes à être positionnées respectivement au-dessus d'un premier passage inférieur de boîte de Petri et au-dessus d'un deuxième passage inférieur de boîtes de Petri, les premier et deuxième passages étant prévus sur une partie fixe par rapport au socle,
un système de déplacement d'au moins une boîte de Petri de la première colonne à la deuxième colonne, comportant :
   - un organe de transfert ayant une ouverture supérieure de support du couvercle d'une boîte de Petri au-dessus d'une deuxième ouverture inférieure de support du fond d'une boîte de Petri,
   - un moyen de distribution pour distribuer ledit produit au-dessus de l'ouverture inférieure dans le fond de la boîte de Petri sous l'ouverture supérieure,
   - l'organe mobile de transfert étant apte à occuper une première position d'arrêt, où les ouvertures de support se trouvent sous le premier passage, et une deuxième position d'arrêt, où les ouvertures de support se trouvent sous le deuxième passage,
   - un premier piston comportant un premier plateau situé sous le premier passage et apte à traverser les ouvertures pour faire descendre le couvercle et le fond d'une boîte de Petri de la première colonne sur leur ouverture respective de support lorsque l'organe de transfert se trouve dans la première position d'arrêt associée,
   - un deuxième piston comportant un deuxième plateau situé sous le deuxième passage inférieur et apte à traverser les ouvertures pour faire remonter le couvercle et le fond de la boîte de Petri de leur ouverture respective de support dans la deuxième colonne, lorsque l'organe de transfert se trouve dans la deuxième position d'arrêt associée,
caractérisé en ce que
l'organe de transfert est une navette à déplacement en translation par rapport au socle selon un mouvement direct aller et retour des ouvertures de support entre l'une et l'autre des première et deuxième positions d'arrêt,
le moyen de distribution étant agencé pour distribuer ledit produit au-dessus de l'ouverture inférieure et sous l'ouverture supérieure dans le fond de la boîte lorsque ces ouvertures se trouvent dans l'une sélectionnée des première et deuxième positions d'arrêt.

Grâce à l'invention, aucun temps n'est perdu pour amener une boîte ouverte auprès d'une station de remplissage en produit

Suivant des modes de réalisation de l'invention :
- Le dispositif comporte des moyens d'entraînement synchrone des pistons à la même hauteur.
- Les pistons sont entraînés par le même moteur.
- Le dispositif comporte des moyens de coordination des déplacements en translation de la navette et des pistons pour les mettre successivement dans les positions suivantes :
- la première position d'arrêt avec une position d'abaissement du premier plateau sous l'ouverture inférieure, pour amener le couvercle de la boîte sur l'ouverture supérieure et amener le fond de la boîte sur l'ouverture inférieure,
- la deuxième position d'arrêt, avec le premier plateau sous l'ouverture inférieure pour la distribution du produit dans le fond de la boîte ouverte située dans l'ouverture inférieure par le moyen de distribution,
- dans la deuxième position d'arrêt, une position d'élévation du deuxième plateau dans la deuxième colonne pour la fermeture du couvercle sur le fond de la boîte et le transfert de cette boîte fermée dans la deuxième colonne,
- la première position d'arrêt avec une position d'abaissement du premier plateau sous l'ouverture inférieure.
- Le moyen de distribution comporte une buse de sortie dudit produit, des moyens de déplacement de la buse de distribution étant prévus pour la faire passer entre l'une et l'autre d'une position de distribution dudit produit, où la buse se trouve au-dessus de l'ouverture inférieure et sous l'ouverture supérieure dans ladite position sélectionnée d'arrêt, et d'une position d'escamotage où la buse ne se trouve plus entre les ouvertures, des moyens de commande étant prévus pour n'autoriser le mouvement du piston associé à la position sélectionnée d'arrêt que lorsque la buse est position d'escamotage.
- La navette comporte en plus desdites ouvertures supérieure et inférieure de support, appelées deuxièmes ouvertures, un moyen de soutien de fond de boîtes de Petri muni d'une première ouverture permettant le traversée du premier piston dans la première position d'arrêt.
- La navette comporte en plus desdites ouvertures supérieure et inférieure de support, appelées deuxièmes ouvertures, des première ouvertures supérieure et inférieure, la navette comporte une platine inférieure et une platine supérieure espacées l'une de l'autre d'une distance prescrite pour ménager un passage du moyen de distribution en position de distribution, la platine inférieure comportant la première ouverture inférieure de traversée du premier plateau et du premier piston et ladite deuxième ouverture inférieure de support du fond de la boîte, la platine supérieure comportant une première ouverture supérieure de traversé du premier plateau et du premier piston et ladite deuxième ouverture supérieure de support du couvercle, la première ouverture supérieure étant au-dessus de la première ouverture inférieure.
- Le dispositif comporte des moyens de montée du premier plateau du premier piston au-dessus de la navette, le premier plateau est fixé à au moins une tige inférieure de liaison du premier plateau auxdits moyens de montée, la navette comporte au moins un passage de communication entre les deuxièmes ouvertures et la première ouverture ou les premières ouvertures pour permettre de laisser passer ladite tige inférieure de soutien lors du déplacement de la navette entre les positions d'arrêt lorsque le premier plateau se trouve en position de montée au-dessus de la navette.
- Le dispositif comporte au moins un carrousel monté rotatif sur un axe vertical du socle, le carrousel comportant une pluralité de colonnes de guidage vertical de boîtes de Petri, aptes à recevoir chacune une pile de boîtes de Petri à couvercle disposé sur le fond, des moyens d'entraînement en rotation du carrousel étant prévus pour amener deux des colonnes respectivement au-dessus du premier passage inférieur de boîte de Petri et au-dessus du deuxième passage inférieur pour former lesdites première et deuxième colonnes.
- Le dispositif comporte des moyens de retenue et de passage de boîtes de Petri au-dessus du deuxième passage, ces moyens de retenue et de passage étant mobiles entre une première position de retenue des boîtes de Petri au-dessus du deuxième passage dans la deuxième colonne et une deuxième position de libération de passage des boîtes de Petri dans le deuxième passage, des moyens de contrainte dans le sens allant de la première position de retenue étant prévus,
des moyens de montée du deuxième plateau du deuxième piston jusqu'à une position prescrite d'élévation dans la deuxième colonne étant prévus,
les moyens de retenue étant agencés pour être actionnés dans la deuxième position de libération de passage par butée contre ledit couvercle de la boîte de Petri lorsque le deuxième plateau du deuxième piston sur lequel se trouve la boîte ayant ce couvercle est monté jusqu'à la position prescrite d'élévation dans la deuxième colonne,
les moyens de retenue ménageant dans la première position de retenue une largeur de passage inférieure à celle du fond de la boîte de Petri pour la soutenir et supérieure à la largeur du deuxième plateau du deuxième piston pour permettre sa descente sous la deuxième colonne.

L'invention sera mieux comprise à la lecture de la description qui va suivre, donnée uniquement à titre d'exemple non limitatif en référence aux dessins annexés, sur lesquels :

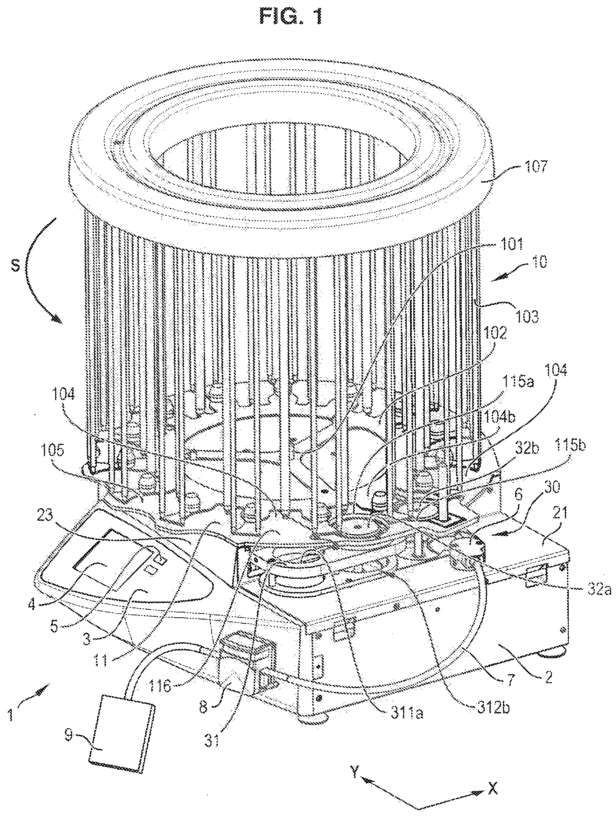

- la figure 1 est une vue schématique en perspective d'un mode de réalisation d'une machine mettant en oeuvre le dispositif de distribution suivant l'invention,
- la figure 2 est une vue schématique en perspective d'une partie du dispositif de la figure 1,
- les figures 3A, 3B, 3C, 3D, 3E, 3F et 3G sont des vues partielles agrandies du dispositif suivant la figure 1 dans différentes positions successives de fonctionnement pour le remplissage d'une boîte de Petri, et
- les figures 4A, 4B, 4C et 4D sont des vues schématiques de profil d'une partie du dispositif suivant la figure 1 dans des positions différentes.

Aux figures, le dispositif 1 de distribution comporte un socle 2 se présentant par exemple sous la forme d'un caisson. Le socle 2 comporte une face avant 22 sur laquelle se trouve un panneau de commande du dispositif, muni par exemple d'un écran de contrôle 4 et d'un ou plusieurs boutons 5 de commande ou toute autre interface 5, permettant à l'utilisateur de commander le fonctionnement automatique du dispositif 1. Sur le socle 2 est prévu un moyen 6 de distribution d'un produit prescrit qui est par exemple de la gélose liquide, ou un milieu gélosé. Ce moyen 6 de distribution est par exemple sous la forme d'une tête 6 rotative autour d'un axe vertical sur une face supérieure 21 du socle 2, la tête 6 comportant une buse 61 de sortie du produit. Ce moyen 6 de distribution est alimenté en produit par un tuyau 7 relié à un moyen 8 d'envoi du produit vers le moyen 6, ce moyen 8 étant par exemple formé par une pompe péristaltique 8 disposée sur le socle 2, par exemple sur sa face avant 22, l'autre extrémité du tuyau 7 étant reliée à une réserve 9 de produit.

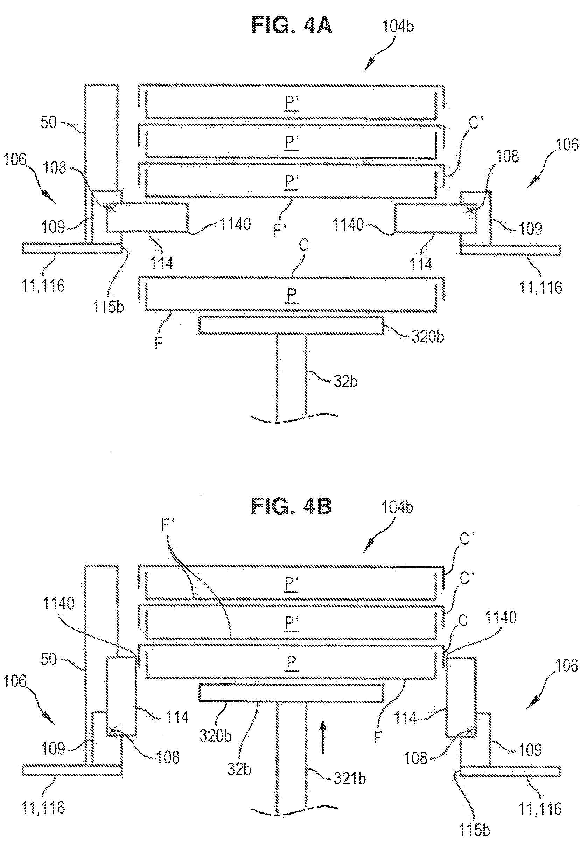

Le socle 2 comporte une partie supérieure 23 surélevée par rapport à la face supérieure latérale 21, adjacente à cette partie supérieure 23. La partie surélevée 23 supporte un carrousel 10 de stockage de boîtes de Petri. Le carrousel 10 est par unique sur le socle 2 et est rotatif par rapport à un axe vertical central 101 sur la partie 23 du socle 2. Le carrousel 10 comporte un anneau inférieur 102 supportant des moyens 103 de stockage de boîtes de Petri en piles. Ces moyens 103 délimitent une pluralité de colonnes verticales 104 de logement et de guidage de piles de boîtes de Petri et comprennent à cet effet des tiges 103 ou tout autre moyen de guidage vertical de boîtes de Petri. Un anneau supérieur 107 amovible est prévu pour coiffer les moyens 103. Chaque colonne 104 débouche vers le bas par une ouverture 105 de passage d'une boîte de Petri dans l'anneau inférieur 102. Par conséquent, ces ouvertures 105 ont un diamètre supérieur à celui des boîtes de Petri. Les ouvertures 105 des colonnes 104 sont réparties à équidistance de l'axe central 101 de rotation et de manière équiangle sur l'anneau inférieur 102. Les ouvertures 105 par exemple périphériques à l'anneau inférieur 102 et sous la forme d'échancrures de l'anneau 102. La hauteur des moyens 103 de guidage est prévu pour la réception dans chaque colonne 104 d'une pile de plusieurs boîtes de Petri en un nombre prescrit jusqu'à l'anneau 107. Chaque boîte P de Petri est introduite dans une colonne 104 en ayant son couvercle C posé sur son fond F pour que la boîte soit fermée de manière amovible, les boîtes P de Petri ayant de manière connu un couvercle C plus large que leur fond F, ainsi que cela est représenté aux figures 4A et 4B. Lorsque la boîte de Petri est fermée, le rebord du couvercle se trouve à l'extérieur du rebord du fond.

Sous l'anneau inférieur 102 du carrousel et sur le socle 2 se trouve une plaque 11 de guidage horizontal des boîtes de Petri lorsqu'elles sont dans le carrousel 10. L'axe 101 du carrousel est relié à des moyens d'entrainement en rotation situés dans le socle 2 pour faire tourner horizontalement le carrousel 10 autour de cet axe vertical 101. La plaque 11 de guidage horizontal est fixée sur la partie surélevée 23 et comporte une partie 116 située au-dessus de la surface supérieure 21 et sous plusieurs colonnes 104 du carrousel 10. La plaque 11 de guidage horizontal comporte dans la partie 116 une première ouverture ou passage 115a et une deuxième ouverture ou passage 115b ayant la même disposition spatiale que deux ouvertures inférieures 105 du carrousel 10, pour permettre le passage d'une boîte de Petri au travers de ces ouvertures 105, 115a, 115b. Pour plus de compacité, l'écartement entre la première ouverture 115a et la deuxième ouverture 115b de la plaque 105 correspond à l'écartement entre deux première et deuxième ouvertures inférieures respectives adjacentes 105a et 105b du carrousel 10.

Les première et deuxième ouvertures 115a et 115b de la plaque 11 sont situées au-dessus d'une station 20 permettant à la fois le dépilement des boîtes de Petri vides, leur ouverture, la distribution du produit dans leur fond, leur fermeture et leur ré-empilement à l'état rempli. La station 20 se situe sur la face supérieure 21 du socle 2.

La station 20 comporte un système 30 de déplacement des boîtes de Petri une à une à l'état vide depuis le dessous de la colonne 104b dans l'ouverture 105b par l'ouverture 115a, par l'intermédiaire d'un organe 31 de transfert en translation horizontale et de deux premier et deuxième pistons 32a, 32b à déplacement vertical sous respectivement les deuxième et première colonnes 104a, 104b. Chaque piston 32a, 32b comporte un plateau supérieur 320a, 320b, fixé à une tige verticale inférieure 321a, 321b reliée dans le socle 2 à des moyens d'entrainement en déplacement vertical pour faire monter et descendre les pistons 22a, 22b.

Suivant l'invention, l'organe 31 de transfert est une navette 31 effectuant un aller-retour en translation suivant une direction horizontale longitudinale X.

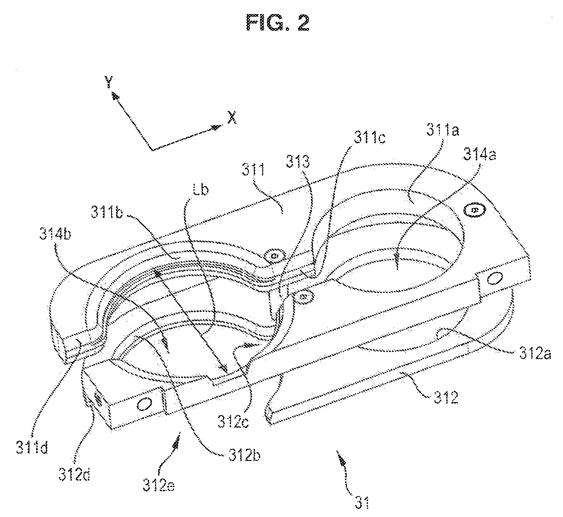

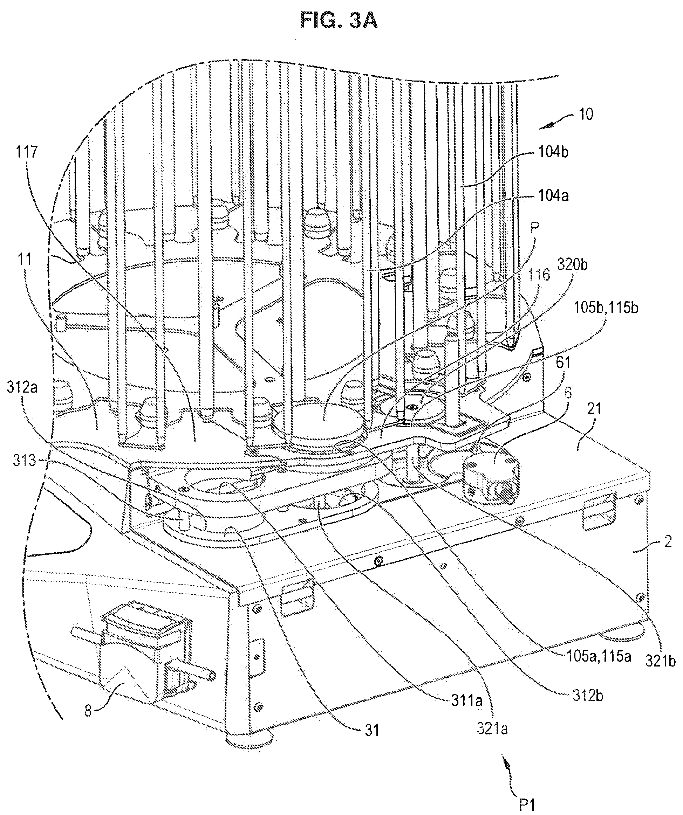

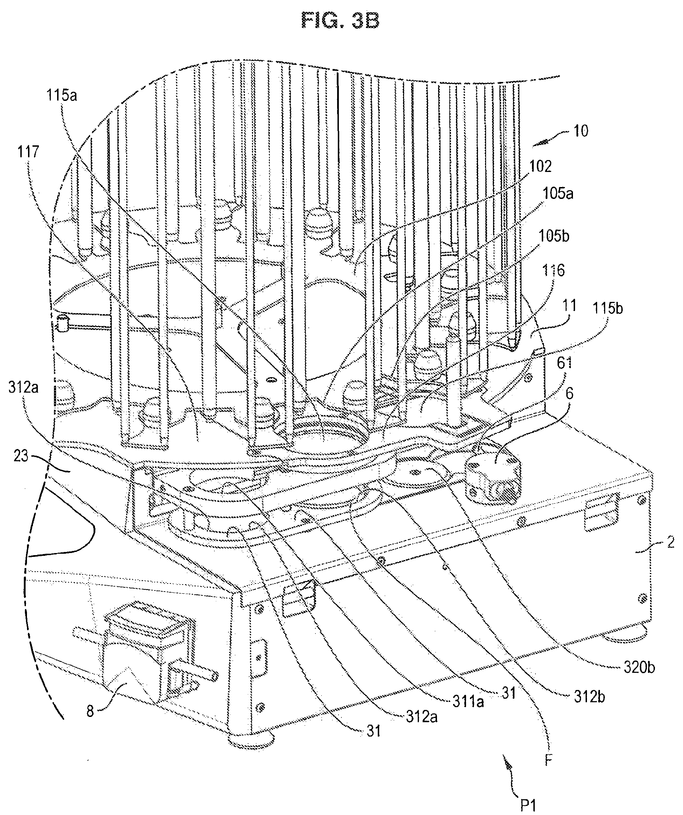

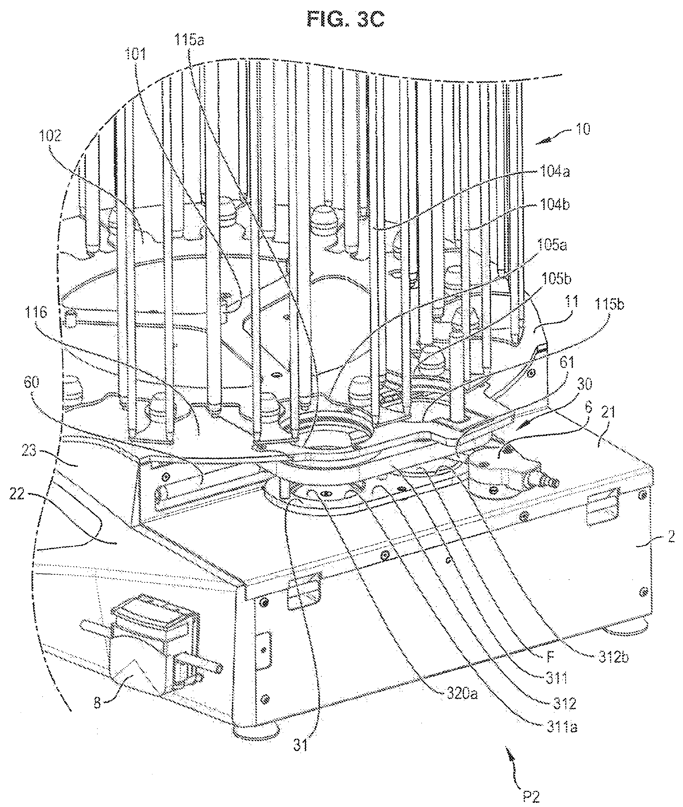

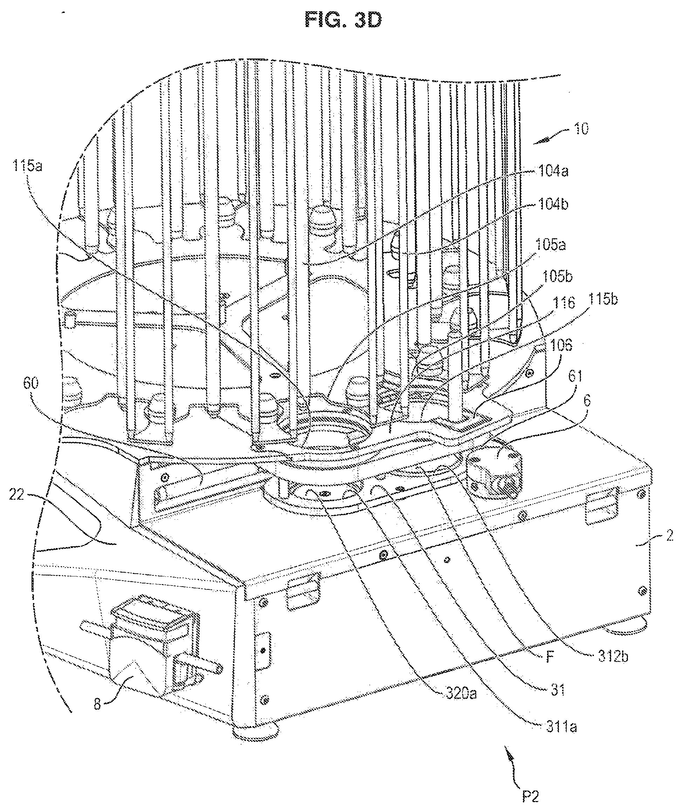

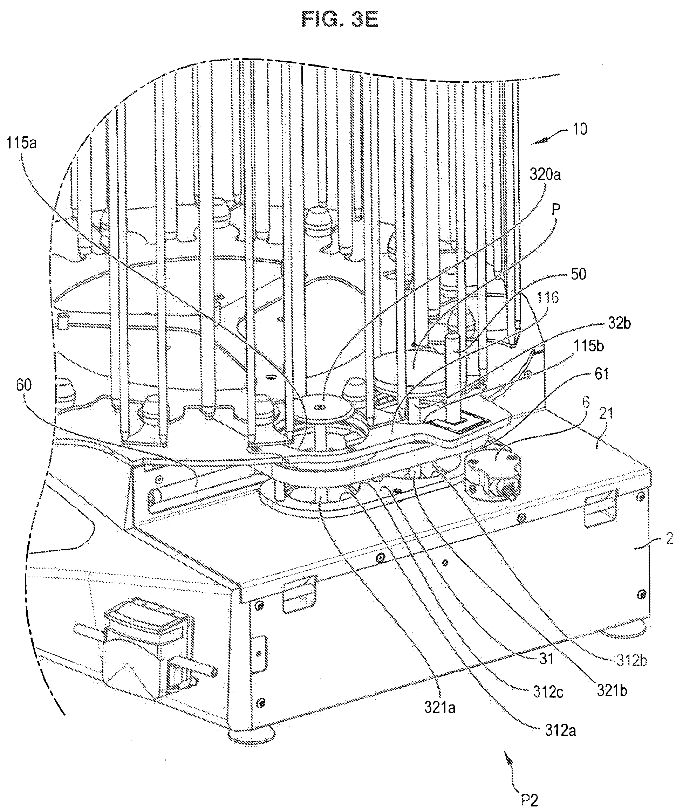

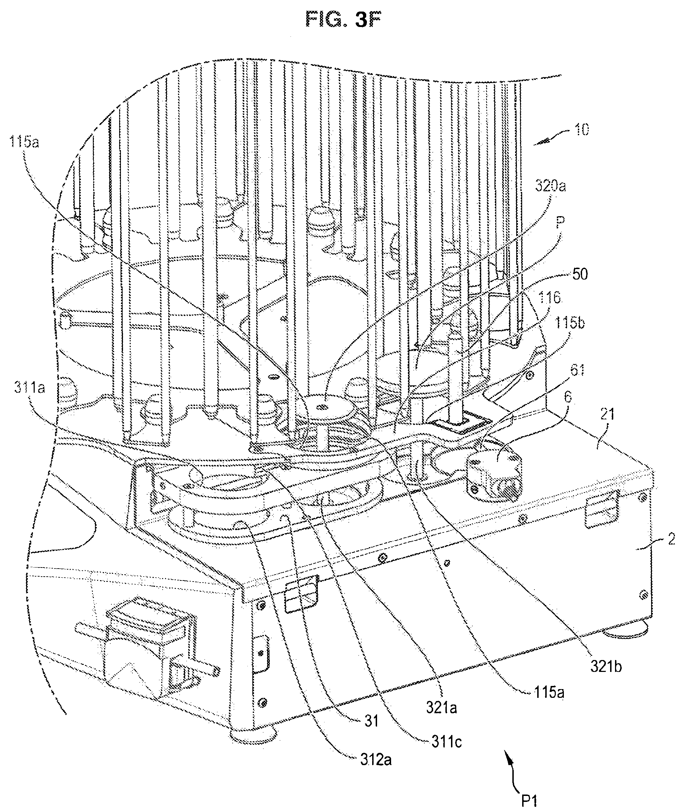

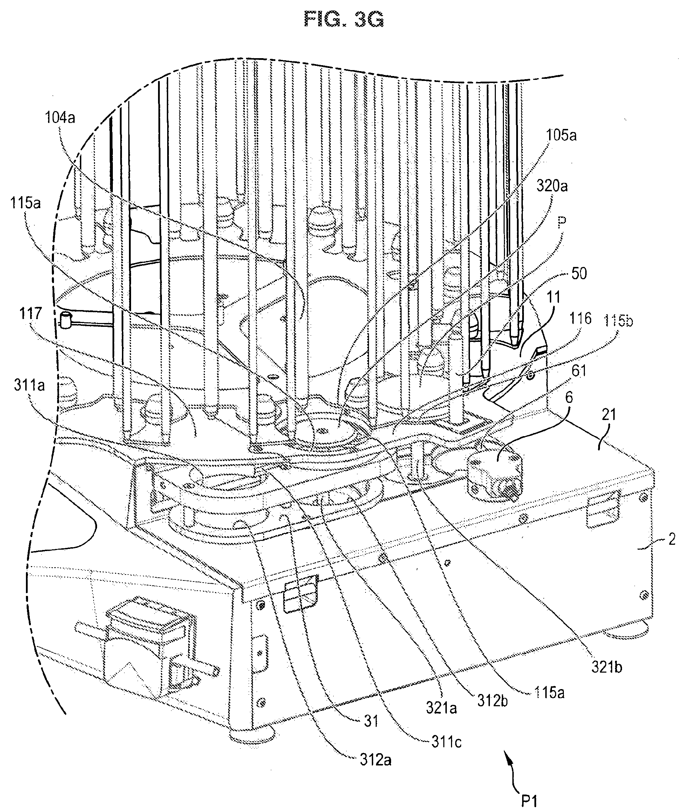

Un mode de réalisation de l'organe 31 est représenté à la figure 2 et est décrit plus en détail ci-dessous. Dans ce mode de réalisation, ainsi que représenté aux figures 3A, 3B, 3C, 3D, 3E, 3F et 3G, les pistons 32a et 32b ont des mouvements de translation verticale simultanée, de manière à ce que leurs plateaux 320a, 320b se trouvent à la même hauteur l'un de l'autre. Ceci permet dans ce cas de n'avoir qu'un seul moteur d'entraînement en translation verticale pour les deux pistons 32a et 32b. Dans le mode de réalisation représenté, les premier et deuxième pistons 32a et 32b présentent le même mouvement en synchronisme, et se trouvent en même temps en position haute (figures 3A, et 3G), en positon basse (figures 3B, 3C, 3D) ou en position d'élévation (figures 3E, 3F).

La navette 31 comporte une platine supérieure 311 et une platine inférieure 312 solidaires l'une de l'autre au moyen d'entretoises 313. Bien entendu, la navette 31 peut être réalisée en une seule pièce. La platine inférieure 312 sert au guidage en translation de la navette 31 sur la surface 21 et à la stabilité de la navette 21 sur cette surface 21. Les moyens 60 d'entraînement en translation de la navette 31 sont latéraux et logés dans le socle 2 sous la partie surélevée 23.

Aux figures, la platine supérieure 311 comporte une première ouverture supérieure 311a et une deuxième ouverture supérieure 311b, qui sont situées respectivement au-dessus d'une première ouverture inférieure 312a et d'une deuxième ouverture inférieure 312b de la platine inférieure 312. Les premières ouvertures inférieure et supérieure 311a et 312a servent au passage du premier plateau 320a du premier piston 32a et sont plus larges que ce dernier. La deuxième ouverture supérieure 311b sert au passage du fond F d'une boîte de Petri et à retenir le couvercle C d'une boîte de Petri. La deuxième ouverture supérieure 311b possède une largeur Lb supérieure à celle du fond F d'une boîte de Petri, mais inférieure à celle de son couvercle C. La deuxième ouverture inférieure 312b sert au support du fond F d'une boîte de Petri et possède une largeur inférieure à celle de ce fond F. Les deuxièmes ouvertures 311b et 312b sont donc appelées ouvertures de support d'une boîte P de Petri.

En outre, la platine supérieure 311 comporte un passage 311c de liaison entre la première ouverture supérieure 311a et la deuxième ouverture supérieure 311b, pour permettre le passage de la tige 321a du premier piston 32a entre ces ouvertures 311a et 311b. Le passage 311 c possède une largeur horizontale transversale supérieure à celle de la tige 321a du premier piston 32a. La platine inférieure 312 comporte un passage 312c de liaison entre la première ouverture inférieure 312a et la deuxième ouverture inférieure 312b, pour permettre le passage de la tige 321a du premier piston 32a entre ces ouvertures 311a et 311b. Le passage 312c possède une largeur horizontale transversale supérieure à celle de la tige 321 a du premier piston 32a.

A la figure 2, la navette 31 s'étend suivant la direction horizontale longitudinale X qui est la direction horizontale allant entre les deux ouvertures 115a et 115b de la plaque 105. La direction horizontale transversale Y est celle perpendiculaire à la direction longitudinale X.

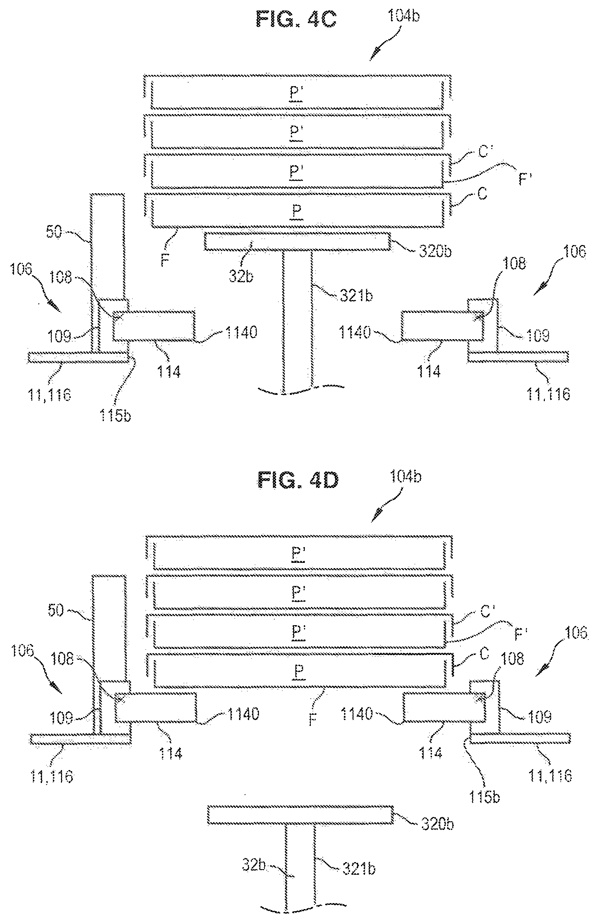

La plaque 11 comporte des moyens 106 de retenue et de passage des boîtes P, P' de Petri empilées au-dessus de la deuxième ouverture 115b. Ces moyens 106 sont mobiles entre une première position de retenue d'une pile de boîte P' de Petri par le dessous au-dessus de l'ouverture 115b dans la colonne 104b, ainsi que cela est représenté aux figures 4A, 4C et 4D, et une deuxième position de libération de passage des boîtes de Petri dans l'ouverture 115b, ainsi que cela est représenté à la figure 4B. Ces boîtes P' se trouvant déjà dans la colonne 104b comportent un couvercle C' posé sur un fond F'. Les moyens 106 comprennent par exemple en plusieurs endroits autour de l'ouverture 115b une patte 114 articulée par un axe 108 de rotation horizontale sur un support 109 fixé à la partie 116 à proximité de l'ouverture 115b, par exemple deux pattes 114 diamétralement opposées. Dans la position de retenue des figures 3B, 3C, 3D et 4A, les pattes 114 s'étendent au-dessus de l'ouverture 115b pour délimiter entre eux une largeur inférieure à celle du fond F' des boîtes de Petri P' pour soutenir le fond F' de celle du bas, cette largeur permettant le passage du plateau 320b du piston 32b entre les pattes 114 et étant supérieure à la largeur de ce plateau 320b.

Lorsque le plateau 320b ayant dessus une boîte P de Petri ayant son couvercle C posé sur son fond F traverse de bas en haut l'ouverture 115b vers la colonne 104b à la figure 4B, le couvercle C de cette boîte B, soulevé par le plateau 320b, soulève les pattes 114 pour les faire tourner vers l'extérieur. Au cours de la montée du plateau 320b à la figure 4B, le plateau 320b est à une hauteur telle par rapport à l'ouverture 115b que les pattes 114 sont en appui par leur extrémité libre 1140 éloignée de l'axe 108 latéralement contre les boîtes P, P' de Petri. Des moyens sont prévus pour contraindre les pattes 114 à être appliquées vers la position de retenue, par exemple par gravité ou du fait du positionnement de leur axe 108 dans la patte 114, ou par un ressort ou autres moyen positif de rappel.

A la figure 4C, le plateau 320b passe dans une position d'élévation au-dessus de l'extrémité libre 1140 qui était appuyée contre les boîtes P, P' soutenues par le plateau. Cette position d'élévation correspond aux figures 3E et 3F. Par conséquent, l'extrémité 1140 des pattes retombe dans la position de retenue vers l'ouverture 115b.

Lorsque de la figure 4C à la figure 4D, le piston 32b redescend, son plateau 320b passe entre les pattes 114 se trouvant en position de retenue, puis traverse l'ouverture 115b, ce qui dépose la pile de boîtes P, P' sur les pattes 114 en position de retenue.

Le fonctionnement du dispositif est le suivant.

On a représenté aux figures 3A, 3B, 3C, 3D, 3E, 3F et 3G le trajet d'une boîte P de Petri vide, c'est-à-dire comportant un couvercle C et un fond F, qui est celle située la plus en bas de la colonne 104a.

Aux figures 3A, 3B, 3F et 3G, la navette 31 se trouve dans une première position d'arrêt.

Aux figures 3C, 3D et 3E, la navette 31 se trouve dans une deuxième position d'arrêt.

Dans la première position P1 d'arrêt, les ouvertures de support 311b et 312b de la navette 31 se trouvent sous la première ouverture 115a, se trouvant elle-même sous une ouverture 105a d'une colonne 104a de boîtes de Petri vides et fermées. Dans la première position P1 d'arrêt, la navette 31 est disposée de l'autre côté du deuxième piston 32b, une partie pleine 117 de la plaque 11 étant interposée entre le carrousel 10 et la première ouverture 311a supérieure de la navette 31.

Dans la deuxième position P2 d'arrêt, les premières ouvertures 311a et 312a de la navette 31 se trouvent sous la première ouverture 115a et sous la première colonne 104a. Dans la deuxième position d'arrêt, les deuxièmes ouvertures 311b et 312b de la navette 31 se trouvent sous la deuxième ouverture 115b et sous la deuxième colonne 104b.

Dans le mode de réalisation représenté aux figures, la navette 31 comporte comme position d'arrêt, uniquement la première position P1 d'arrêt et la deuxième position P2 d'arrêt.

A la figure 3A, le premier plateau 320a du premier piston 32a soutient la première pile 104a de boîtes P de Petri dans la première colonne 104a au-dessus des deuxièmes ouvertures 312b et 311b, la tige 321a passant dans ces ouvertures 312b et 311b.

Puis, le piston 32a est abaissé au-travers des deuxièmes ouvertures 311b et 312b de la navette 31 pour amener le couvercle C de la boîte P de Petri du bas de la colonne 104a sur la deuxième ouverture supérieure 311b et pour amener le fond F de la boîte P sur la deuxième ouverture inférieure 312b, pour arriver dans la position représentée à la figure 3B. Aux figures 3B, 3C, 3D, les plateaux 310a, 320b des pistons se trouvent sous la navette 31, par exemple dans des logements ménagés dans la surface 21.

Aux figures 3A et 3B, le moyen 6 de distribution de produit se trouve dans une position d'escamotage où la buse 61 ne se trouve pas au-dessus de l'ouverture 312b ni au-dessus du piston 32b. Dans cette position d'escamotage, l'embout 61 laisse libre le passage entre la deuxième ouverture inférieure 312b et la deuxième ouverture supérieure 311b et se trouve dans ce cas en dehors de la course du piston 32b.

Puis, la navette 31 est déplacée par translation horizontale longitudinale suivant la direction X pour arriver dans la deuxième position d'arrêt représentée à la figure 3C. Le moyen 6 de distribution passe à une position de distribution pour le déversement du produit, par exemple par rotation de la tête 36 de distribution de manière à amener son embout 61 de déversement entre les platines 311 et 312 au-dessus de la deuxième ouverture inférieure 312b et donc au-dessus du fond F s'y trouvant à la figure 3C.

Le fond F supporté sur/dans la deuxième ouverture 312b et le couvercle C supporté sur/dans la deuxième ouverture supérieure 311b se trouvent sous la deuxième ouverture 115b de la plaque fixe 11 et sous la deuxième colonne 104b. Des moyens automatiques prévus dans le socle 2 provoquent alors la commande du moyen 8 d'envoi pour faire parvenir du produit dans le moyen 6 de distribution, ce produit étant alors déversé par ce moyen 6 au-dessus de la deuxième ouverture supérieure 312b dans le fond F de boîte de Petri s'y trouvant.

Puis, dans la position représentée à la figure 3D, le moyen 6 de distribution est remis dans sa position écartée de la deuxième ouverture inférieure 312b ou position d'escamotage.

Puis, à la figure 3E, les pistons 32a et 32b passent dans la position d'élévation de la figure 4C au-dessus de la navette 31 et dans la colonne 104b. Le deuxième piston 32b soulève donc d'abord le fond F se trouvant dans la deuxième ouverture inférieure 312b, puis remonte le fond F dans le couvercle C se trouvant dans la deuxième ouverture supérieure 311b, ce qui ferme la boîte P de Petri dont le fond contient du produit ayant été déverse dedans par le moyen 6, puis soulève la boîte P ainsi fermée et remplie dans la deuxième colonne 104b au travers de la deuxième ouverture 115b et de la deuxième ouverture 104b du carrousel 10. Le plateau 320b soutient donc la boîte P fermée et remplie dans la colonne 104b et les autres boîtes P' qui se trouvaient éventuellement déjà dans la colonne 104b.

Ensuite, à la figure 3F, la navette 31 est translatée dans sa première position d'arrêt correspondant à la figure 3B. A la figure 3F, les deuxièmes ouvertures 312b et 311b de la navette 31 se trouvent sous les premières ouvertures 115a et 104a, tandis que les premières ouvertures 311a et 312a se trouvent sous la partie pleine 117 de la plaque 11. Au cours de ce mouvement de translation, la tige 321a du premier piston 32a passe donc des deuxièmes ouvertures 311b et 312b aux 311a et 312a en traversant les passages 311c et 312c ménagés dans les platines 311 et 312.

Puis, à la figure 3G, les plateaux 320a et 320b des pistons reviennent à une position plus basse que la position d'élévation de la figure 3F, cette position de la figure 3G correspondant à celle des figure 3A et 4D. Aux figures 3A et 3G, les boîtes de Petri de la colonne 104b sont soutenues par les moyens 106.

Un moyen 50 de guidage vertical des boîtes P, P' dans la deuxième colonne 104b est fixé sur la plaque 11 à proximité de l'ouverture 115b, et comprend par exemple une barre verticale 50 pour mettre les boîtes P, P' suivant un empilement droit lors de la montée et de la descente du deuxième piston 32b aux figures 4A à 4D.

L'encombrement de la navette 31 est d'environ deux boîtes de Petri juxtaposées correspondant à son positionnement sous deux colonnes 104 adjacentes comme à la figure 3C. La course de la navette 31 correspond dans le mode de réalisation représenté à la distance entre les deux colonnes 104a et 104b de boîtes de Petri adjacentes.

La largeur de la première ouverture supérieure 311a est plus petite que la largeur du fond F d'une boîte de Petri P pour soutenir la pile de boîtes de Petri issues de la première colonne 104a lorsque cette première ouverture supérieure 311a se trouve dessous aux figures 3C et 3D.

Bien entendu, la navette 31 pourrait avoir toute autre forme que celle représentée aux figures, tout en ayant un premier passage vertical 314a allant de la première ouverture inférieure 312a à la première ouverture supérieure 311a pour permettre le déplacement du premier plateau 320a du premier piston 32a dans celui-ci, un deuxième passage vertical 314b allant de la deuxième ouverture inférieure 312b à la deuxième ouverture supérieure 311b pour le passage des premier et deuxième plateaux 320a et 320b des premier et deuxième piston 32a et 32b et le troisième passage 311c supérieur entre les ouvertures supérieures 311b et 31 la et le quatrième passage inférieur 312c entre les ouvertures inférieures 312a et 312b, ces troisième et quatrième passages 311 c et 311 d pouvant bien entendu n'être qu'un.

Du fait du faible encombrement de la navette 31, il est possible de réaliser un carrousel 10 ayant un plus grand nombre de colonnes 104. Par conséquent, à nombre de boîtes P de Petri égal, on peut arriver à un carrousel 10 moins haut, du fait de boîtes de Petri réparties sur un plus grand nombre de colonnes 104. La fabrication du dispositif est également plus simple et moins coûteuse.

Pour une utilisation optimale du dispositif, l'utilisateur remplit toutes les colonnes 104 du carrousel sauf une de piles de boîtes de Petri vides. La colonne vide de boites de Petri est amenée par rotation du carrousel 10 au-dessus de la deuxième ouverture 115b et au-dessus du plateau 320b du deuxième piston 32 b à la figure 3A. Puis on remplit successivement les boîtes P de Petri vides issues de la colonne 104a selon le procédé décrit ci-dessus.

Les boîtes P remplies sont donc transférées l'une après l'autre dans la colonne 104b ne comportant initialement aucune boite. Lorsque la colonne 104a située au-dessus de la première ouverture 115a ne comporte plus de boîtes P, on fait tourner le carrousel 10 dans le sens S représenté à la figue 1 pour faire passer cette nouvelle colonne vide au-dessus de la deuxième ouverture 115b. Ce sens S d'avancée du carrousel d'une colonne va dans le même sens que de la première position P1 d'arrêt à la deuxième position P2 d'arrêt. Dans ce cas, une nouvelle colonne de boîtes de Petri vides, qui était initialement sur la partie pleine 112 de la plaque 11, se retrouve au-dessus de la première ouverture 115a de dépilement. On recommence alors le même processus avec cette pile de boîtes.

La navette 31 comporte également un passage d'extrémité longitudinal pour la tige 321b du piston, c'est-à-dire dans le mode de réalisation représentée, un premier passage d'extrémité longitudinal supérieur 311d dans la platine supérieure 311, débouchant d'une part sur la deuxième ouverture supérieure 311b et d'autre part sur l'extérieur dans le sens longitudinal et d'autre part un passage d'extrémité longitudinal inférieur 312d dans la platine inférieure 312, faisant communiquer la deuxième ouverture inférieure 312b avec l'extérieur dans le sens longitudinal. Par exemple, à la figure 2, la deuxième ouverture inférieure 312b et le passage 312d sont sous la forme d'une échancrure de la platine inférieure 312 débouchant vers l'extrémité longitudinale inférieure et vers un côté 312e.

Un détecteur de présence d'un fond F dans la deuxième ouverture inférieure 312b peut être prévu. Ce détecteur est par exemple fixe par rapport au socle 2 pour détecter la présence du fond F dans la deuxième ouverture inférieure 312b dans la première position d'arrêt et/ou dans la deuxième position d'arrêt. Il peut donc être prévu un premier détecteur sous la première colonne 104a. Il peut être également prévu un autre détecteur sous la deuxième colonne 104b.Le processus de remplissage des boîtes P est effectué tant que le détecteur fournit une information de présence de fond F de boîte dans l'ouverture 312b. Dans le cas où le détecteur ne fournit pas une information de présence de fond F de boîte dans l'ouverture 312b, le processus est interrompu ou l'envoi de produit dans le moyen 6 de distribution est interrompu.

Les moyens de commande et de coordination, pour commander les différents moyens d'entraînement du carrousel 10, des pistons 32a, 32b et de la navette 31, sont automatisés et actionnés par l'interface 5 de commande. Ils sont prévus par exemple sous la forme d'un microprocesseur dans le socle 2.

Le dispositif suivant l'invention est destiné à être intégré à un automate de remplissage de boîtes de Petri.

## Revendications

1. Dispositif de distribution d'un produit prescrit dans au moins une boîte de Petri, chaque boîte (P) de Petri comportant un couvercle (C) amovible apte à être disposé sur un fond (F) moins large que celui-ci, le dispositif comportant sur un socle (2) :
au moins des première et deuxième colonnes (104a, 104b) de guidage vertical de boîtes (P) de Petri, aptes à recevoir respectivement des première et deuxième piles de boîtes de Petri à couvercle disposé sur le fond et aptes à être positionnées respectivement au-dessus d'un premier passage inférieur (115a) de boîte de Petri et au-dessus d'un deuxième passage inférieur (115b) de boîtes de Petri, les premier et deuxième passages (115a, 115b) étant prévus sur une partie fixe (3) par rapport au socle (2),
un système (30) de déplacement d'au moins une boîte de Petri de la première colonne (104a) à la deuxième colonne (104b), comportant :
- un organe (31) de transfert ayant une ouverture supérieure (311b) de support du couvercle (C) d'une boîte de Petri au-dessus d'une deuxième ouverture inférieure (312b) de support du fond (F) d'une boîte de Petri,
- un moyen (6) de distribution pour distribuer ledit produit au-dessus de l'ouverture inférieure (312b) dans le fond (F) de la boîte de Petri sous l'ouverture supérieure (311b),
- l'organe (31) mobile de transfert étant apte à occuper une première position d'arrêt, où les ouvertures (311b, 312b) de support se trouvent sous le premier passage (115a), et une deuxième position d'arrêt, où les ouvertures (311b, 312b) de support se trouvent sous le deuxième passage (115b),
- un premier piston (32a) comportant un premier plateau (320a) situé sous le premier passage (115a) et apte à traverser les ouvertures (311b, 312b) pour faire descendre le couvercle et le fond d'une boîte de Petri de la première colonne (104a) sur leur ouverture respective (311b, 312b) de support lorsque l'organe (31) de transfert se trouve dans la première position d'arrêt associée,
- un deuxième piston (32b) comportant un deuxième plateau (320b) situé sous le deuxième passage inférieur (115b) et apte à traverser les ouvertures (311b, 312b) pour faire remonter le couvercle et le fond de la boîte de Petri de leur ouverture respective (311b, 312b) de support dans la deuxième colonne (104b), lorsque l'organe (31) de transfert se trouve dans la deuxième position d'arrêt associée,
**caractérisé en ce que**
l'organe (31) de transfert est une navette (31) à déplacement en translation par rapport au socle (2) selon un mouvement direct aller et retour des ouvertures (311b, 312b) de support entre l'une et l'autre des première et deuxième positions (P1, P2) d'arrêt,
le moyen (6) de distribution étant agencé pour distribuer ledit produit au-dessus de l'ouverture inférieure (312b) et sous l'ouverture supérieure (311b) dans le fond (F) de la boîte (P) lorsque ces ouvertures (311b, 312b) se trouvent dans l'une sélectionnée des première et deuxième positions (P1, P2) d'arrêt.

2. Dispositif suivant la revendication 1, **caractérisé en ce qu'**il comporte des moyens d'entraînement synchrone des pistons (32a, 32b) à la même hauteur.

3. Dispositif suivant la revendication 2, **caractérisé en ce que** les pistons (32a, 32b) sont entraînés par le même moteur.

4. Dispositif suivant l'une quelconque des revendications précédentes, **caractérisé en ce que** le dispositif comporte des moyens de coordination des déplacements en translation de la navette (31) et des pistons (32a, 32b) pour les mettre successivement dans les positions suivantes :
- la première position (P1) d'arrêt avec une position d'abaissement du premier plateau (320a) sous l'ouverture inférieure (312b), pour amener le couvercle de la boîte sur l'ouverture supérieure (311b) et amener le fond de la boîte sur l'ouverture inférieure (312b),
- la deuxième position (P2) d'arrêt, avec le premier plateau (320a) sous l'ouverture inférieure (312b) pour la distribution du produit dans le fond (F) de la boîte ouverte située dans l'ouverture inférieure (312b) par le moyen (6) de distribution,
- dans la deuxième position (P2) d'arrêt, une position d'élévation du deuxième plateau (320b) dans la deuxième colonne (104b) pour la fermeture du couvercle sur le fond de la boîte et le transfert de cette boîte fermée dans la deuxième colonne,
- la première position (P1) d'arrêt avec une position d'abaissement du premier plateau (320a) sous l'ouverture inférieure (312b).

5. Dispositif suivant l'une quelconque des revendications précédentes, **caractérisé en ce que** le moyen (6) de distribution comporte une buse (61) de sortie dudit produit, des moyens de déplacement de la buse (6) de distribution étant prévus pour la faire passer entre l'une et l'autre d'une position de distribution dudit produit, où la buse (6) se trouve au-dessus de l'ouverture inférieure (312b) et sous l'ouverture supérieure (311b) dans ladite position sélectionnée d'arrêt, et d'une position d'escamotage où la buse (6) ne se trouve plus entre les ouvertures (311b, 312b), des moyens de commande étant prévus pour n'autoriser le mouvement du piston (32a, 32b) associé à la position sélectionnée d'arrêt que lorsque la buse (6) est position d'escamotage.

6. Dispositif suivant l'une quelconque des revendications précédentes, **caractérisé en ce que** la navette (31) comporte en plus desdites ouvertures (311b, 312b) supérieure et inférieure de support, appelées deuxièmes ouvertures (311b, 312b), un moyen (311b) de soutien de fond (F) de boîtes de Petri muni d'une première ouverture (311b) permettant le traversée du premier piston (32a) dans la première position d'arrêt.

7. Dispositif suivant l'une quelconque des revendications précédentes, **caractérisé en ce que** la navette (31) comporte en plus desdites ouvertures (311b, 312b) supérieure et inférieure de support, appelées deuxièmes ouvertures (311b, 312b), des première ouvertures (311a, 312a) supérieure et inférieure, la navette (31) comporte une platine inférieure (312) et une platine supérieure (311) espacées l'une de l'autre d'une distance prescrite pour ménager un passage du moyen (6) de distribution en position de distribution, la platine inférieure (312) comportant la première ouverture inférieure (312a) de traversée du premier plateau (320a) et du premier piston (32a) et ladite deuxième ouverture inférieure (312b) de support du fond de la boîte, la platine supérieure (311) comportant une première ouverture supérieure (311a) de traversé du premier plateau (320a) et du premier piston (32a) et ladite deuxième ouverture supérieure (311b) de support du couvercle, la première ouverture supérieure (311a) étant au-dessus de la première ouverture inférieure (312a).

8. Dispositif suivant l'une quelconque des revendications 6 et 7, **caractérisé en ce qu'**il comporte des moyens de montée du premier plateau (320a) du premier piston (32a) au-dessus de la navette (31), le premier plateau (320a) est fixé à au moins une tige inférieure (321a) de liaison du premier plateau (320a) auxdits moyens de montée, la navette (31) comporte au moins un passage (311c, 312c) de communication entre les deuxièmes ouvertures (311b, 312b)et la première ouverture (311 a) ou les premières ouvertures (311 a, 312a) pour permettre de laisser passer ladite tige inférieure (321a) de soutien lors du déplacement de la navette (31) entre les positions (P1, P2) d'arrêt lorsque le premier plateau (320a) se trouve en position de montée au-dessus de la navette (31).

9. Dispositif suivant l'une quelconque des revendications précédentes, **caractérisé en ce qu'**il comporte au moins un carrousel (10) monté rotatif sur un axe vertical du socle (2), le carrousel comportant une pluralité de colonnes (104a, 104b) de guidage vertical de boîtes (P) de Petri, aptes à recevoir chacune une pile de boîtes de Petri à couvercle disposé sur le fond, des moyens d'entraînement en rotation du carrousel (10) étant prévus pour amener deux des colonnes (104a, 104b) respectivement au-dessus du premier passage inférieur (115a) de boîte de Petri et au-dessus du deuxième passage inférieur (115b) pour former lesdites première et deuxième colonnes (104a, 104b).

10. Dispositif suivant l'une quelconque des revendications précédentes, **caractérisé en ce qu'**il comporte des moyens (106) de retenue et de passage de boîtes (P, P') de Petri au-dessus du deuxième passage (115b), ces moyens (106) de retenue et de passage étant mobiles entre une première position de retenue des boîtes (P, P') de Petri au-dessus du deuxième passage (115b) dans la deuxième colonne (104b) et une deuxième position de libération de passage des boîtes de Petri dans le deuxième passage (115b), des moyens de contrainte dans le sens allant de la première position de retenue étant prévus,
des moyens de montée du deuxième plateau (320b) du deuxième piston (32b) jusqu'à une position prescrite d'élévation dans la deuxième colonne (104b) étant prévus,
les moyens (106) de retenue étant agencés pour être actionnés dans la deuxième position de libération de passage par butée contre ledit couvercle de la boîte de Petri lorsque le deuxième plateau (320b) du deuxième piston (32b) sur lequel se trouve la boîte ayant ce couvercle est monté jusqu'à la position prescrite d'élévation dans la deuxième colonne (104b),
les moyens (106) de retenue ménageant dans la première position de retenue une largeur de passage inférieure à celle du fond de la boîte de Petri pour la soutenir et supérieure à la largeur du deuxième plateau (320b) du deuxième piston (32b) pour permettre sa descente sous la deuxième colonne (104b).

## Patentansprüche

1. Vorrichtung zur Verteilung eines verschriebenen Produktes in mindestens einer Petri-Schale, wobei jede Petri-Schale (P) eine abnehmbare Abdeckung (C) umfasst, die geeignet ist, auf einem Boden (F) angeordnet zu werden, der weniger breit ist als diese, wobei die Vorrichtung auf einem Sockel (2) Folgendes umfasst:
mindestens eine erste und eine zweite Säule (104a, 104b) zur vertikalen Führung von Petri-Schalen (P), die geeignet sind, erste beziehungsweise zweite Stapel von Petri-Schalen mit auf dem Boden angeordneter Abdeckung aufzunehmen, und geeignet sind, über einem ersten unteren Durchgang (115a) für Petri-Schalen beziehungsweise über einem zweiten unteren Durchgang (115b) für Petri-Schalen positioniert zu werden, wobei der erste und der zweite Durchgang (115a, 115b) auf einem in Bezug zum Sockel (2) festen Teil (3) vorgesehen sind,
ein System (30) zur Verschiebung von mindestens einer Petri-Schale von der ersten Säule (104a) zur zweiten Säule (104b), das Folgendes umfasst:
- ein Übertragungsglied (31), das eine obere Öffnung (311b) zur Unterstützung der Abdeckung (C) einer Petri-Schale über einer zweiten unteren Öffnung (312b) zur Unterstützung des Bodens (F) einer Petri-Schale aufweist,
- ein Verteilungsmittel (6) zum Verteilen des Produktes über der unteren Öffnung (312b) im Boden (F) der Petri-Schale unter der oberen Öffnung (311b),
- wobei das bewegliche Übertragungsglied (31) geeignet ist, eine erste Ruhestellung, in der die Unterstützungsöffnungen (311b, 312b) sich unter dem ersten Durchgang (115a) befinden, und eine zweite Ruhestellung einzunehmen, in der die Unterstützungsöffnungen (311b, 312b) sich unter dem zweiten Durchgang (115b) befinden,
- einen ersten Kolben (32a), der einen ersten Deckel (320a) umfasst, der sich unter dem ersten Durchgang (115a) befindet und geeignet ist, die Öffnungen (311b, 312b) zu durchqueren, um die Abdeckung und den Boden einer Petri-Schale von der ersten Säule (104a) auf ihrer entsprechenden Unterstützungsöffnung (311b, 312b) abzusenken, wenn das Übertragungsglied (31) sich in der ersten zugehörigen Ruhestellung befindet,
- einen zweiten Kolben (32b), der einen zweiten Deckel (320b) umfasst, der sich unter dem zweiten unteren Durchgang (115b) befindet und geeignet ist, die Öffnungen (311b, 312b) zu durchqueren, um die Abdeckung und den Boden der Petri-Schale von ihrer entsprechenden Unterstützungsöffnung (311b, 312b) in der zweiten Säule (104b) anzuheben, wenn das Übertragungsglied (31) sich in der zweiten zugehörigen Ruhestellung befindet, **dadurch gekennzeichnet, dass**:
das Übertragungsglied (31) eine Schiebebuchse (31) mit in Bezug zum Sockel (2) translatorischer Verschiebung gemäß einer direkten Hin- und Rückbewegung der Unterstützungsöffnungen (311b, 312b) zwischen der einen und der anderen von der ersten und der zweiten Ruhestellung (P1, P2) ist,
das Verteilungsmittel (6) angeordnet ist, um das Produkt über der unteren Öffnung (312b) und unter der oberen Öffnung (311b) im Boden (F) der Schale (P) zu verteilen, wenn diese Öffnungen (311b, 312b) sich in einer ausgewählten von der ersten und der zweiten Ruhestellung (P1, P2) befinden.

2. Vorrichtung nach Anspruch 1, **dadurch gekennzeichnet, dass** sie Mittel zum synchronen Antrieb der Kolben (32a, 32b) auf der gleichen Höhe umfasst.

3. Vorrichtung nach Anspruch 2, **dadurch gekennzeichnet, dass** die Kolben (32a, 32b) durch denselben Motor angetrieben werden.

4. Vorrichtung nach einem der vorhergehenden Ansprüche, **dadurch gekennzeichnet, dass** die Vorrichtung Mittel zur Koordinierung der translatorischen Verschiebungen der Schiebebuchse (31) und der Kolben (32a, 32b) umfasst, um sie nacheinander in die folgenden Stellungen zu bringen:
- die erste Ruhestellung (P1) mit einer Absenkungsstellung des ersten Deckels (320a) unter der unteren Öffnung (312b), um die Abdeckung der Schale auf die obere Öffnung (311b) zu bringen und den Boden der Schale auf die untere Öffnung (312b) zu bringen,
- die zweite Ruhestellung (P2), in der sich der erste Deckel (320a) unter der unteren Öffnung (312b) für die Verteilung des Produktes im Boden (F) der offenen Schale, die sich in der unteren Öffnung (312b) befindet, durch das Verteilungsmittel (6) befindet,
- in der zweiten Ruhestellung (P2) eine Anhebungsstellung des zweiten Deckels (320b) in der zweiten Säule (104b) zum Schließen der Abdeckung auf dem Boden der Schale und zum Übertragen dieser geschlossenen Schale in die zweite Säule,
- die erste Ruhestellung (P1) mit einer Absenkungsstellung des ersten Deckels (320a) unter der unteren Öffnung (312b).

5. Vorrichtung nach einem der vorhergehenden Ansprüche, **dadurch gekennzeichnet, dass** das Verteilungsmittel (6) eine Düse (61) für den Austritt des Produktes umfasst, wobei Mittel zur Verschiebung der Verteilungsdüse (6) vorgesehen sind, um sie zwischen der einen und der anderen von einer Stellung zur Verteilung des Produktes, in der die Düse (6) sich über der unteren Öffnung (312b) und unter der oberen Öffnung (311b) in der ausgewählten Ruhestellung befindet, und einer eingezogenen Stellung übergehen zu lassen, in der die Düse (6) sich nicht mehr zwischen den Öffnungen (311b, 312b) befindet, wobei Steuerungsmittel vorgesehen sind, um die Bewegung des zugehörigen Kolbens (32a, 32b) in die ausgewählte Ruhestellung lediglich zuzulassen, wenn die Düse (6) sich in der eingezogenen Stellung befindet.

6. Vorrichtung nach einem der vorhergehenden Ansprüche, **dadurch gekennzeichnet, dass** die Schiebebuchse (31) zusätzlich zu den oberen und unteren Unterstützungsöffnungen (311b, 312b), die zweite Öffnungen (311b, 312b) genannt werden, ein Mittel (311b) zum Stützen des Bodens (F) der Petri-Schalen umfasst, das mit einer ersten Öffnung (311b) versehen ist, die die Durchführung des ersten Kolbens (32a) in der ersten Ruhestellung ermöglicht.

7. Vorrichtung nach einem der vorhergehenden Ansprüche, **dadurch gekennzeichnet, dass** die Schiebebuchse (31) zusätzlich zu den oberen und unteren Unterstützungsöffnungen (311b, 312b), die zweite Öffnungen (311b, 312b) genannt werden, erste obere und untere Öffnungen (311a, 312a) umfasst, wobei die Schiebebuchse (31) eine untere Platine (312) und eine obere Platine (311) umfasst, die voneinander in einem vorgeschriebenen Abstand beabstandet sind, um einen Durchgang des Verteilungsmittels (6) in der Verteilungsstellung einzurichten, wobei die untere Platine (312) die erste untere Öffnung (312a) zur Durchführung des ersten Deckels (320a) und des ersten Kolbens (32a) und die zweite untere Öffnung (312b) zur Unterstützung des Bodens der Schale umfasst, wobei die obere Platine (311) eine erste obere Öffnung (311a) für die Durchführung des ersten Deckels (320a) und des ersten Kolbens (32a) und die zweite obere Öffnung (311b) zur Unterstützung der Abdeckung umfasst, wobei die erste obere Öffnung (311a) sich über der ersten unteren Öffnung (312a) befindet.

8. Vorrichtung nach einem der Ansprüche 6 und 7, **dadurch gekennzeichnet, dass** sie Mittel zum Anheben des ersten Deckels (320a) des ersten Kolbens (32a) über der Schiebebuchse (31) umfasst, wobei der erste Deckel (320a) an mindestens einer unteren Stange (321a) zur Verbindung des ersten Deckels (320a) an den Anhebemitteln befestigt ist, wobei die Schiebebuchse (31) mindestens einen Durchgang (311b, 312b) zur Verbindung zwischen den zweiten Öffnungen (311b, 312b) und der ersten Öffnung (311a) oder der ersten Öffnungen (311a, 312a) umfasst, um den Durchgang der unteren Stützstange (321a) bei der Verschiebung der Schiebebuchse (31) zwischen den Ruhestellungen (P1, P2) zu ermöglichen, wenn der erste Deckel (320a) sich in der Anhebungsstellung über der Schiebebuchse (31) befindet.

9. Vorrichtung nach einem der vorhergehenden Ansprüche, **dadurch gekennzeichnet, dass** sie mindestens einen Kreisförderer (10) umfasst, der drehbar auf einer vertikalen Achse des Sockels (2) angebracht ist, wobei der Kreisförderer mehrere Säulen (104a, 104b) zur vertikalen Führung von Petri-Schalen (P) umfasst, die geeignet sind, jeweils einen Stapel von Petri-Schalen mit auf dem Boden angeordneter Abdeckung aufzunehmen, wobei Mittel zum drehbaren Antrieb des Kreisförderers (10) vorgesehen sind, um zwei der Säulen (104a, 104b) über den ersten unteren Petri-Schalen-Durchgang (115a) und über den zweiten unteren Durchgang (115b) zu bringen, um die erste und die zweite Säule (104a, 104b) zu bilden.

10. Vorrichtung nach einem der vorhergehenden Ansprüche, **dadurch gekennzeichnet, dass** sie Mittel (106) zum Halten und für den Durchgang von Petri-Schalen (P, P') über dem zweiten Durchgang (115b) umfasst, wobei diese Halte- und Durchgangsmittel (106) zwischen einer ersten Stellung zum Halten der Petri-Schalen (P, P') über dem zweiten Durchgang (115b) in der zweiten Säule (104b) und einer zweiten Stellung zur Freigabe des Durchgangs der Petri-Schalen in dem zweiten Durchgang (115b) beweglich sind, wobei Mittel zur Spannung in die Richtung vorgesehen sind, die von der ersten Haltestellung verläuft,
Mittel zum Anheben des zweiten Deckels (320b) des zweiten Kolbens (32b) bis zu einer vorgeschriebenen Höhenstellung in der zweiten Säule (104b) vorgesehen sind,
die Haltemittel (106) angeordnet sind, um in der zweiten Stellung zur Freigabe des Durchgangs durch Anschlag gegen die Abdeckung der Petri-Schale betätigt zu werden, wenn der zweite Deckel (320b) des zweiten Kolbens (32b), auf dem sich die Schale befindet, die diese Abdeckung aufweist, bis zu der vorgeschriebenen Höhenstellung in der zweiten Säule (104b) angehoben ist,
wobei die Haltemittel (106) in der ersten Haltestellung eine Durchgangsbreite einrichten, die kleiner als diejenige des Bodens der Petri-Schale, um sie zu unterstützen, und größer ist als die Breite des zweiten Deckels (320b) des zweiten Kolbens (32b), um ihr Absenken unter die zweite Säule (104b) zu ermöglichen.

## Claims

1. Device for dispensing a prescribed product into at least one Petri dish (P), each Petri dish (P) comprising a removable lid (C) able to be arranged over a bottom part (F) of smaller width than the lid, the device comprising on a base (2):
- at least first and second columns (104a, 104b) for the vertical guiding of Petri dishes (P), respectively capable of receiving first and second stacks of Petri dishes with lids placed over the bottom part and capable of being positioned respectively above a first lower passageway (115a) for Petri dishes and above a second lower passageway (115b) for Petri dishes, the first and second passageways (115a, 115b) being provided on a fixed part (3) relative to the base (2),
- a system (30) for moving at least one Petri dish from the first column (104a) to the second column (104b) comprising:
- a transfer member (31) having an upper opening (311b) supporting the lid (C) of a Petri dish above a second lower opening (312b) supporting the bottom part (F) of a Petri dish,
- dispensing means (6) to dispense the said product above the lower opening (312b) into the bottom part (F) of the Petri dish underneath the upper opening (311b),
- the mobile transfer member (31) being capable of taking up a first stop position in which the supporting openings (311b, 312b) are located under the first passageway (115a), and a second stop position in which the supporting openings (311b, 312b) are located under the second passageway (115b),
- a first piston (32a) comprising a first tray (320a) located under the first passageway (115a) and capable of passing through the openings (311b, 312b) to lower the lid and the bottom part of a Petri dish in the first column (104a) onto their respective supporting openings (311b, 312b) when the transfer member (31) lies in the associated first stop position,
- a second piston (32b) comprising a second tray (320b) located under the second lower passageway (115b) and capable of passing through the openings (311b, 312b) to lift the lid and bottom part of the Petri dish from their respective supporting openings (311b, 312b) up into the second column (104b) when the transfer member (31) lies in the associated second stop position, **characterized in that**:
- the transfer member (31) is a shuttle (31) with translational movement relative to the base (2) in direct back and forth movement of the supporting openings (311b, 312b) between each of the first and second stop positions (P1, P2),
- the dispensing means (6) being arranged to dispense the said product above the lower opening (312b) and under the upper opening (311b) into the bottom part (F) of the Petri dish (P) when these openings (311b, 312b) lie in a selected one of the first and second stop positions (P1, P2).

2. The device according to claim 1, **characterized in that** it comprises means for synchronous driving of the pistons (32a, 32b) at the same height.

3. The device according to claim 2, **characterized in that** the pistons (32a, 32b) are driven by the same motor.

4. The device according to any of the preceding claims, **characterized in that** the device comprises means for coordinating the translational movements of the shuttle (31) and pistons (32a, 32b) to place them successively in the following positions:
- the first stop position (P1) with a lowered position of the first tray (320a) under the lower opening (312b) to bring the lid of the dish onto the upper opening (311b) and to bring the bottom of the dish onto the lower opening (312b),
- the second stop position (P2) with the first tray (320a) under the lower opening (312b) for dispensing of the product by the dispensing means (6) into the bottom part (F) of the open dish located in the lower opening (312b),
- in the second stop position (P2), an elevation position of the second tray (320b) in the second column (104b) to close the lid on the bottom of the dish and to transfer the closed dish to the second column,
- the first stop position (P1) with a lowered position of the first tray (320a) under the lower opening (312b).

5. The device according to any of the preceding claims, **characterized in that** the dispensing means (6) comprise an outlet nozzle (61) for said product, means to move the dispensing nozzle (6) being provided to cause it to move between one and the other of a dispensing position of said product in which the nozzle (6) lies above the lower opening (312b) and under the upper opening (311b) in said selected stop position, and a retracted position in which the nozzle (6) no longer lies between the openings (311b, 312b), control means being provided so as only to allow movement of the piston (32a, 32b) associated with the selected stop position when the nozzle (6) is in retracted position.

6. The device according to any of the preceding claims, **characterized in that** the shuttle (31), in addition to said upper and lower supporting openings (311b, 312b) called second openings (311b, 312b), comprises means (311b) for supporting the bottom part (F) of Petri dishes having a first opening (311b) to allow passing of the first piston (32a) in the first stop position.

7. The device according to any of the preceding claims, **characterized in that** the shuttle (31), in addition to said upper and lower supporting openings (311b, 312b) called second openings (311b, 312b), comprises first upper and lower openings (311a, 312a), the shuttle (31) comprises a bottom-plate (312) and a top-plate (311) spaced apart by a prescribed distance to arrange a passageway for the dispensing means (6) in the dispensing position, the bottom-plate (312) comprising the first lower opening (312a) for passing of the first tray (320a) and of the first piston (32a) and said second lower opening (312b) supporting the bottom part of the dish, the top-plate (311) comprising a first upper opening (311a) for passing of the first tray (320a) and of the first piston (32a) and said second upper opening (311b) supporting the lid, the first upper opening (311a) being positioned above the first lower opening (312a).

8. The device according to either of claims 6 and 7, **characterized in that** it comprises means for lifting the first tray (320a) of the first piston (32a) to above the shuttle (31), the first tray (320a) is fixed to at least one lower rod (321a) linking the first tray (320a) to said lifting means, the shuttle (31) comprises at least one passageway (311c, 312c) communicating the second openings (311b, 312b) with the first opening (311a) or first openings (311a, 312a) to allow passing of the lower supporting rod (321a) when the shuttle (31) moves between the stop positions (P1, P2) and the first tray (320a) is in raised position above the shuttle (31).

9. The device according to any of the preceding claims, **characterized in that** it comprises at least one carousel (10) mounted in rotation on a vertical axis of the base (2), the carousel comprising a plurality of columns (104a, 104b) for vertical guiding of the Petri dishes (P) and each capable of receiving a stack of Petri dishes with lids arranged over the bottom part, means for driving the carousel (10) in rotation being provided to bring two of the columns (104a, 104b) respectively above the first lower passageway (115a) for Petri dishes and above the second lower passageway (115b) to form said first and second columns (104a, 104b).

10. The device according to any of the preceding claims, **characterized in that** it comprises means (106) for retaining and passing Petri dishes (P, P') above the second passageway (115b), these retaining and passing means (106) being mobile between a first retaining position of the Petri dishes (P, P') above the second passageway (115b) in the second column (104b), and a second release position allowing passing of the Petri dishes in the second passageway (115b), provision being made for constraining means to constraint to the first retaining position,
- means being provided for lifting the second tray (320b) of the second piston (32b) up to a prescribed elevation position in the second column (104b),
- the retaining means (106) being arranged to be actuated in the second release position, by abutment against said lid of the Petri dish when the second tray (320b) of the second piston (32b) carrying the dish associated with the lid is lifted to the prescribed elevation position in the second column (104b),
the retaining means (106) forming a passageway in the first retaining position which is less wide than the width of the bottom part of the Petri dish to give support thereto and which is wider than the width of the second tray (320b) of the second piston (32b) to allow lowering thereof under the second column (104b).
